# EUROPÄISCHE PATENTANMELDUNG

(11) **EP 1 120 424 A2**
(43) Veröffentlichungstag der Anmeldung: **01.08.2001**
(21) Anmeldenummer: 01100145.0
(22) Anmeldetag: 16.01.2001
(51) Int. Cl.: C07F 17/00, C07F 7/12, C08F 10/00

(54) **Metallorganische Verbindungen mit anellierten Indenyl Liganden**

(30) Priorität: 28.01.2000 DE 10003581
(71) Anmelder: BAYER AG, 51368 Leverkusen (DE)
(72) Erfinder: Becke, Sigurd, Dr., 51503 Rösrath (DE); Lang, Heinrich, Prof. Dr., 09125 Chemnitz (DE); Weiss, Thomas, 68256 Mannheim (DE)

(57) **Zusammenfassung**

Die vorliegende Erfindung betrifft metallorganische Verbindungen von Übergangsmetallen mit einem in 2-Position gebundenen und in 5,6-Position anellierten Indenyl-Liganden, ein Verfahren zu ihrer Herstellung und ihre Verwendung als Katalysatoren zur (Co)Polymerisation von olefinischen und/oder diolefinischen Monomeren.

## Beschreibung

Die vorliegende Erfindung betrifft metallorganische Verbindungen von Übergangsmetallen mit einem in 2-Position gebundenen und in 5,6-Position anellierten Indenyl-Liganden, ein Verfahren zu ihrer Herstellung und ihre Verwendung als Katalysatoren zur (Co)Polymerisation von olefinischen und/oder diolefinischen Monomeren.

Entprechend der IUPAC-Nomenklatur werden die Positionen der Ringatome des Indens in der vorliegenden Anmeldung wie folgt bezeichnet:

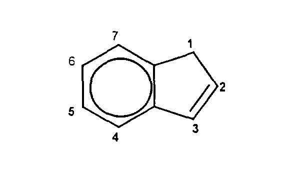

Stereorigide chirale Metallocene mit verbrückten Indenylliganden als Katalysatoren zur Herstellung von Polyolefinen sind bekannt. Dabei hat sich erwiesen, dass Art und Position der Substituenten am Indenylanion und Art und Position der Verbrückung einen Einfluß sowohl auf die Katalysatoraktivität, als auch auf die Polymereigenschaften besitzt. Viele der Indenyl-Metallocene weisen eine Verbrückung in 1-Position auf (1-Indenyl-Metallocene).

Besondere Bedeutung besitzen die in 2 und/oder 4 Position substituierten Bis(1-indenyl)-Metallocene mit in 1-Position verbrückten Indenylresten zur Herstellung von hochisotaktischem Polypropylen mit hoher Kristallinität und hohem Schmelzpunkt. (EP-A1-485 821, EP-A1-485 823, EP-A2-519237). Ebenso von Bedeutung sind die in 4,5-Position benzanellierten Bis(1-indenyl)-Metallocene (siehe Organometallics 1994, 13, 964-970).

Es ist auch bekannt, metallorganische Verbindungen mit nur einem Indenylanion als Katalysatoren einzusetzen (Constrained Geometry Komplexe mit 1-Indenylliganden, siehe US-A-5, 026, 798,WO-97/15583-A1).

Aus WO-94/11 406-A1 sind metallorganische Verbindungen von Übergangsmetallen bekannt, die einen Indenyl- und einen Cyclopentadienyl-Liganden aufweisen, wobei der Indenyl-Ligand in der 2-Position substituiert ist; dieser Substituent kann auch als Brücke zum 2. Liganden ausgebildet sein. Die Ausführungsbeispiele zeigen vielstufige Herstellungen mit äußerst unbefriedigenden Ausbeuten, die bei verbrückten Verbindungen zu 1-Cyclopentadienyl-2-(2-indenyl)-ethan-zirkoniumchlorid, zu Bis-(2-indenyl)-methan-zirkoniumdichlorid oder zu Dimethyl-bis-(2-indenyl)-silan-zirkoniumdichlorid, welches noch Verunreinigungen enthält, führen. In Organometallics 1993, 12, 5012-5015 wird ein mehrstufiger Syntheseweg zu Ethylenbis(2-indenyl)titandichlorid beschrieben. Aufgrund der vielstufigen Synthese und der zahlreichen Reinigungsoperationen ist die erzielbare Ausbeute sehr gering. Bedingt durch den Syntheseweg ist die Strukturvielfalt auf Ethylen-verbrückte Liganden eingeschränkt.

In EP-A2-941 997 werden Ethylen-verbrückte Bis(2-indenyl)zirkonocene offengelegt. Diese Zirkonocene werden zur Herstellung von speziellen Polyolefinen mit niedrigen Molekulargewichten eingesetzt.

In EP-A1-940 408 werden silylverbrückte 2-Indenyl-Metallocene und ein Verfahren zur Herstellung von metallorganischen Verbindungen mit einem in 2-Position gebundenen Indenyl-Liganden beschrieben.

Über metallorganische Verbindungen mit in 5,6-Position anellierten Indenyl-Liganden (z. B. Tetrahydroindacenyl-Liganden) ist vergleichsweise wenig bekannt. In WO-98/09999-A1 wird in Beispiel 3 die Herstellung eines Halbsandwich-Titankomplexes mit einem Tetrahydroindacenyl-Liganden offengelegt. Die Zugänglichkeit des in 1-Position verbrückten Tetrahydroindacenyl-Titankomplexes ist jedoch unbefriedigend (Gesamtausbeute < 1 %). In WO-98/49212-A1 und WO-98/27103-A1 wird die Herstellung von Halbsandwich-Komplexen mit in 1-Position verbrückten und in 2 und/oder 3 Position substituierten Tetrahydroindacenyl-Liganden und deren Verwendung als Katalysatoren zur Polymerisation von Olefinen beschrieben.

Übergangsmetallkomplexe mit in 2-Position verbrückten Tetrahydroindacenyl-Liganden sind nicht bekannt.

Es hat sich nun gezeigt, dass solche metallorganische Katalysatoren, deren Verbrückung an der 2-Position mindestens eines Tetrahydroindacenylanions ansetzt, besondere Eigenschaften als Polymerisationskatalysatoren haben; sie erzeugen nämlich bei der (Co)Polymerisation von α-Olefinen weitgehend ataktische Polymere mit hohen Molekulargewichten. Es war daher wünschenswert, ein Herstellungsverfahren für solche in der 2-Position mindestens eines Tetrahydroindacenylanions verbrückte Katalysatoren zu finden.

Eine weitere Aufgabe bestand darin, einen Katalysator zur Verfügung zu stellen, der für die Synthese von hochmolekularem EPDM geeignet ist.

Die vorliegende Erfindung betrifft ein Verfahren zur Herstellung von metallorganischen Verbindungen von Übergangsmetallen mit in 5,6-Position anellierten 2-Indenyl als erstem Liganden der Formel 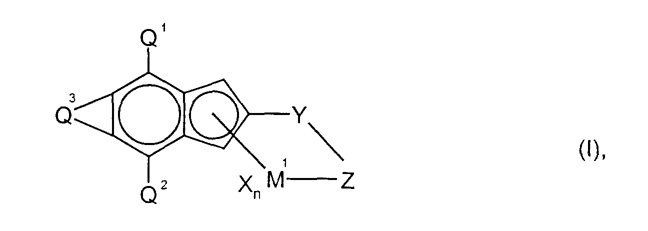 worin
- Q¹, Q²: gleich oder verschieden sind und als Substituent des in 5,6-Position anellierten 2-Indenylsystems Wasserstoff, C₁-C₄-Alkyl, C₆-C₁₄-Aryl, C₇-C₁₀-Aralkyl, C₁-C₄-Alkoxy, C₁-C₄-Alkylthio, Phenoxy, Phenylthio, Di-C₁-C₄-alkyl-amino, C₆-C₁₄-Aryl-C₁-C₄-alkyl-amino, Di-C₆-C₁₄-arylamino, Dibenzylamino, Tri-C₁-C₄-alkyl-silyl, Di-C₁-C₄-alkyl-boranyl, Phenyl-C₁-C₄-alkyl-boranyl, Diphenylboranyl, Di-C₁-C₄-alkyl-phosphoryl, Diphenylphosphoryl oder Phenyl-C₁-C₄-alkyl-phosphoryl bedeuten,
- Q³: einen gegebenfalls substituierten Alkylenrest darstellt, der zusammen mit den beiden Kohlenstoffatomen des Indenylrestes in 5 und 6 Position ein Ringsystem bildet,
- M¹: ein Übergangsmetall aus der 4., 5. oder 6. Gruppe des Periodensystems der Elemente nach IUPAC 1985 ist,
- X: ein Anion bedeutet,
- n: eine Zahl von Null bis Vier ist, die sich aus der Valenz und dem Bindungszustand von M¹ ergibt,
- Y: eine Brücke aus der Gruppe von -C(R¹R²)-, -Si(R¹R²)-, -Ge(R¹R²)-, -C(R¹R²)-C(R³R⁴)-, -C(R¹R²)-Si(R³R⁴)- oder -Si(R¹R²)-Si(R³R⁴)- darstellt, worin R¹, R², R³ und R⁴ unabhängig voneinander Wasserstoff, Halogen, geradkettiges oder verzweigtes C₁-C₁₀-Alkyl, C₅-C₈-Cycloalkyl, C₆-C₁₄-Aryl oder C₇-C₁₀-Aralkyl bedeuten, und
- Z: ein zweiter Ligand aus der Gruppe von offenkettigen und cyclischen, gegebenenfalls anionischen π-Systemen, -N(R⁵)-, -P(R⁶)-, | N(R⁵R⁷)-, | P(R⁶R⁸)-, -O-, -S-, | OR⁵- oder | SR⁵- ist, wobei der senkrechte Strich links vom Elementsymbol N, P, O bzw. S ein Elektronenpaar bedeutet und die Bindung zwischen Z und M¹ ionischen, kovalenten oder koordinativen Charakter hat und worin R⁵, R⁶, R⁷ und R⁸ unabhängig voneinander den Bedeutungsumfang von R¹ bis R⁴ haben und R⁵ und R⁷ zusätzlich -Si(R¹R²R³) bedeuten können und R⁶ und R⁸ zusätzlich -Si(R¹R²R³), -OR¹, -SR¹ oder -N(R¹R²) bedeuten können,
dadurch gekennzeichnet, dass man ein in 5,6 Position anelliertes Halogeninden der Formel 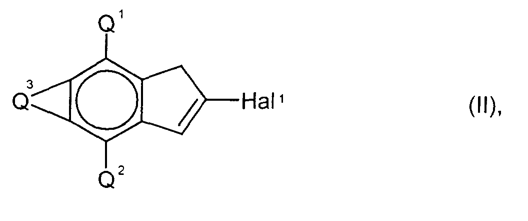 in der Hal¹ für Cl, Br oder I steht und Q¹, Q² und Q³ die obige Bedeutung haben,
mit einem elementaren Metall ausgewählt aus der 1., 2. oder 12. Gruppe des Periodensystems nach IUPAC 1985 oder einer entsprechenden Metallverbindung in einer Menge im Bereich von 1 bis 100 Mol elementares Metall/Metallverbindung pro Mol (II) mit einem Dihalogenid der Brücke Y der Formel

Hal² - Y - Hal³ (III),

in der
- Hal² und Hal³: unabhängig voneinander Cl, Br oder I bedeuten und
- Y: den obigen Bedeutungsumfang hat,
in einer Menge von 1 bis 20 Mol (III) pro Mol (II) umsetzt, wobei in dem Fall, dass Y die Bedeutung -Si(R¹R²)-, -Ge(R¹R²)- oder -Si(R¹R²)-Si(R³R⁴)- hat, die Umsetzung von (II) mit (i) elementares Metall/Metallverbindung und (ii) mit (III) auch simultan erfolgen kann, und das Reaktionsprodukt der Formel 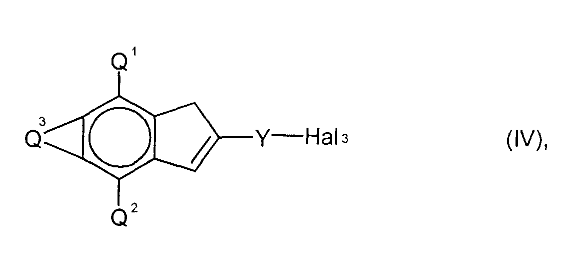 worin Q¹, Q², Q³, Y und Hal³ die obige Bedeutung haben,
gegebenenfalls nach seiner Isolierung mit einem Z-Derivat der Formel

ZM² ₚ (Va)

oder

ZR⁹ ₚ (Vb),

in welcher
- M²: für Li, Na, K oder -MgHal⁴, worin Hal⁴ den Bedeutungsumfang von Hal² hat, steht,
- p: die Zahl Eins oder Zwei darstellt,
- R⁹: Wasserstoff, -Si(R¹R²R³) oder Sn(R¹R²R³) darstellt und
- Z, R¹, R² und R³: die obige Bedeutung haben,
unter Austritt einer Verbindung der Formel

M²Hal³ (VIa)

bzw.

R⁹Hal³ (VIb),

in welcher M², R⁹ und Hal³ die obige Bedeutung haben,
gegebenenfalls in Gegenwart einer Hilfsbase zur 2-Indenyl-Verbindung der Formel 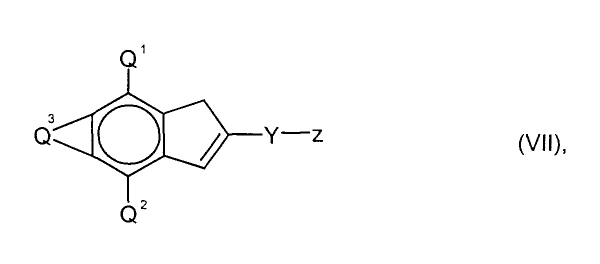 in der Q¹, Q², Q³, Y und Z die obige Bedeutung haben und die als Dianion vorliegen kann und in der Z weiterhin M², R⁹ oder ein Elektronenpaar tragen kann,
und dann weiter mit einer Übergangsmetallverbindung der Formel

M¹X_{q} (VIII),

umsetzt, in der
- M¹ und X: die obige Bedeutung haben und
- q: eine Zahl von Zwei bis Sechs ist, die sich aus der Oxidationsstufe von M¹ ergibt.

Das Verfahren wird vorteilhaft bei Temperaturen im Bereich von -100 bis 120°C durchgeführt.

Als Metalle der Gruppen 1, 2 oder 12 seien insbesondere Lithium, Kalium, Natrium, Magnesium, Calcium, Zink, Cadmium und Quecksilber genannt. Bevorzugt werden die Metalle der Gruppen 2 und 12. Es kann auch vorteilhaft sein, die Metalle in einem Gemisch untereinander einzusetzen.

Als entsprechende Metallverbindungen seien Butyl-Lithium, Butadien-Magnesium Anthracen-Magnesium sowie die entsprechenden Verbindungen der anderen genannten Metalle genannt.

Es kann vorteilhaft sein, die nicht umgesetzte Metalle/Metallverbindungen vor Zugabe von (III) abzutrennen.

In der Regel werden bei Umsetzung mit (III) die entsprechenden Metallhalogenide Metall Hal¹Hal² gebildet.

Weiterhin werden im Regelfall bei Zugabe von (Va) oder (Vb) die entsprechenden Verbindungen der Formeln

M²Hal³ (VIa)

bzw.

R⁹Hal (VIb),

in welchen
- M², R⁹ und Hal³: die bekannten Bedeutungen haben,
gebildet.

Die Erfindung betrifft weiterhin die mit dem genannten Verfahren herstellbaren metallorganischen Verbindungen von Übergangsmetallen mit in 5,6-Position anellierten 2-Indenyl als erstem Liganden der Formel 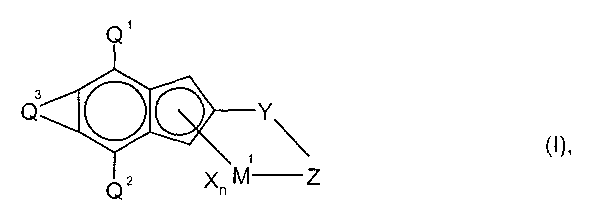 worin
- Q¹, Q²: gleich oder verschieden sind und als Substituent des in 5,6-Position anellierten 2-Indenylsystems Wasserstoff, C₁-C₄-Alkyl, C₆-C₁₄-Aryl, C₇-C₁₀-Aralkyl, C₁-C₄-Alkoxy, C₁-C₄-Alkylthio, Phenoxy, Phenylthio, Di-C₁-C₄-alkyl-amino, C₆-C₁₄-Aryl-C₁-C₄-alkyl-amino, Di-C₆-C₁₄-arylamino, Dibenzylamino, Tri-C₁-C₄-alkyl-silyl, Di-C₁-C₄-alkyl-boranyl, Phenyl-C₁-C₄-alkyl-boranyl, Diphenylboranyl, Di-C₁-C₄-alkyl-phosphoryl, Diphenylphosphoryl oder Phenyl-C₁-C₄-alkyl-phosphoryl bedeuten,
- Q³: einen gegebenfalls substituierten Alkylenrest darstellt, der zusammen mit den beiden Kohlenstoffatomen des Indenylrestes in 5 und 6 Position ein Ringsystem bildet,
- M¹: ein Übergangsmetall aus der 4., 5. oder 6. Gruppe des Periodensystems der Elemente nach IUPAC 1985 ist,
- X: ein Anion bedeutet,
- n: eine Zahl von Null bis Vier ist, die sich aus der Valenz und dem Bindungszustand von M¹ ergibt,
- Y: eine Brücke aus der Gruppe von -C(R¹R²)-, -Si(R¹R²)-, -Ge(R¹R²)-, -C(R¹R²)-C(R³R⁴)-, -C(R¹R²)-Si(R³R⁴)- oder -Si(R¹R²)-Si(R³R⁴)- darstellt, worin R¹, R², R³ und R⁴ unabhängig voneinander Wasserstoff, Halogen, geradkettiges oder verzweigtes C₁-C₁₀-Alkyl, C₅-C₈-Cycloalkyl, C₆-C₁₄-Aryl oder C₇-C₁₀-Aralkyl bedeuten, und
- Z: ein zweiter Ligand aus der Gruppe von offenkettigen und cyclischen, gegebenenfalls anionischen π-Systemen, -N(R⁵)-, -P(R⁶)-, | N(R⁵R⁷)-, | P(R⁶R⁸)-, -O-, -S-, | OR⁵- oder | SR⁵- ist, wobei der waagerechte Strich links vom Elementsymbol N, P, O bzw. S eine kovalente Bindung zwischen Z und M¹ darstellt und wobei der senkrechte Strich links vom Elementsymbol N, P, O bzw. S ein Elektronenpaar bedeutet und die Bindung zwischen Z und M¹ koordinativen (nicht kovalenten) Charakter hat und worin R⁵, R⁶, R⁷ und R⁸ unabhängig voneinander den Bedeutungsumfang von R¹ bis R⁴ haben und R⁵ und R⁷ zusätzlich -Si(R¹R²R³) bedeuten können und R⁶ und R⁸ zusätzlich -Si(R¹R²R³), -OR¹, -SR¹ oder -N(R¹R²) bedeuten können.

Bevorzugt sind Verbindungen der Formel 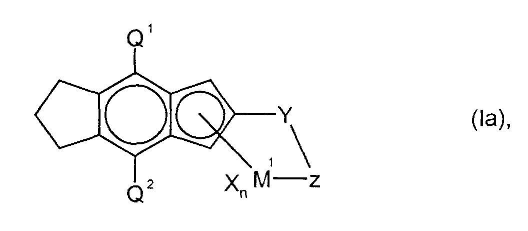 worin Q¹, Q², Y, Z, X, M¹ und n die obige Bedeutung haben.

Das erfindungsgemäße Verfahren ist gekennzeichnet durch eine Reaktionsfolge über das Zwischenprodukt der obigen Formel (IV). Solche Zwischenprodukte sind bislang nicht bekannt. Die Erfindung betrifft daher weiter diese Zwischenprodukte.

Die Erfindung betrifft noch weiter ein Verfahren zur Herstellung der Zwischenprodukte der Formel (IV), das dadurch gekennzeichnet ist, dass man ein in 5,6-Position anelliertes 2-Halogeninden der Formel 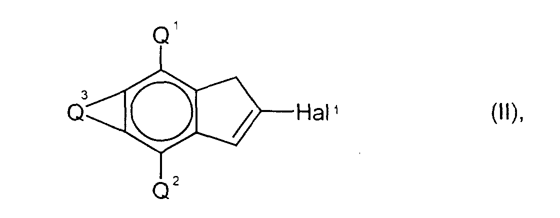 in der
- Hall, Q¹, Q² und Q³: die obige Bedeutung haben,
mit einem elementaren Metall ausgewählt aus der 1., 2. oder 12. Gruppe des Periodensystems der Elemente nach IUPAC 1985 oder einer entsprechenden Metallverbindung in einer Menge im Bereich von 1 bis 100 Mol elementares Metall/Metallverbindung pro Mol (II) und mit einem Dihalogenid von Y der Formel

Hal²-Y-Hal³ (III),

in der
- Y, Hal² und Hal³: die obige Bedeutung haben,
in einer Menge von 1 bis 20 Mol (III) pro Mol (II) umsetzt, wobei in dem Fall, dass Y die Bedeutung -Si(R¹R²)-, -Ge(R¹R²)- oder -Si(R¹R²)-Si(R³R⁴)- hat, die Umsetzung von (II) mit (i) dem elementaren Metall/Metallverbindung und (ii) mit (III) auch simultan erfolgen kann.

Als Metalle der Gruppen 1, 2 oder 12 seien insbesondere Lithium, Kalium, Natrium, Magnesium, Calcium, Zink, Cadmium und Quecksilber genannt. Bevorzugt werden die Metalle der Gruppen 2 und 12. Es kann auch vorteilhaft sein, die Metalle in einem Gemisch untereinander einzusetzen.

Als entsprechende Metallverbindungen seien Butyl-Lithium, Butadien-Magnesium Anthracen-Magnesium sowie die entsprechenden Verbindungen der anderen genannten Metalle genannt.

Es kann vorteilhaft sein, die nicht umgesetzte Metalle/Metallverbindungen vor Zugabe von (III) abzutrennen.

In der Regel werden bei Umsetzung mit (III) die entsprechenden Metallhalogenide Metall Hal¹Hal² gebildet.

Weiterhin werden im Regelfall bei Zugabe von (Va) oder (Vb) die entsprechenden Verbindungen der Formeln

M²Hal³ (VIa)

bzw.

R⁹Hal (VIb),

in welchen
- M², R⁹ und Hal³: die bekannten Bedeutungen haben
gebildet.

Das Verfahren wird vorteilhaft bei Temperaturen im Bereich von -100°C bis +120°C durchgeführt.

Die Erfindung betrifft noch weiter ein Verfahren zur Herstellung der Zwischenprodukte der Formel (II), das dadurch gekennzeichnet ist, dass man das gegebenenfalls substituierte Indanon der Formel 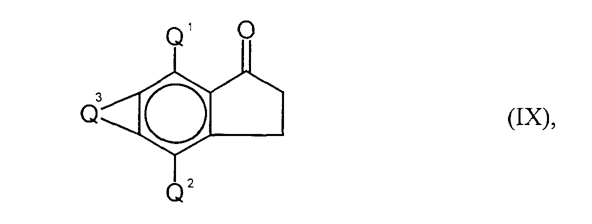 durch Umsetzung der aromatischen Verbindung der Formel 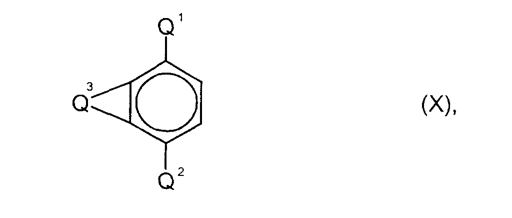 mit einem Acrylsäurederivat der Formel 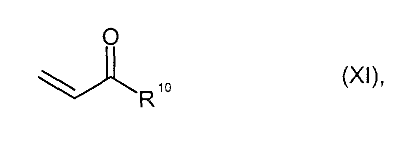 wobei
- R¹⁰: Cl, Br, I, eine Hydroxygruppe oder eine C₁-C₁₀-Alkoxygruppe bedeutet,
in Gegenwart einer Lewissäure herstellt,
wobei als Lewissäure bevorzugt AlCl₃, SbCl₅, FeCl₃, SnCl₄, ZnCl₂ oder BF₃ geeignet ist,
anschließend nach der in J. Organomet. Chem. 568 (1998) 41-51 (Beispiel 3.10) beschriebenen Methode weiter zu einem in 5,6-Position anellierten Inden der Formel 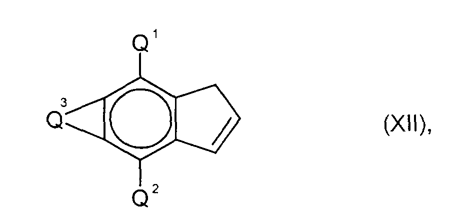 umsetzt,
und dann weiter in das Dihalogenderivat (XIII) überführt, 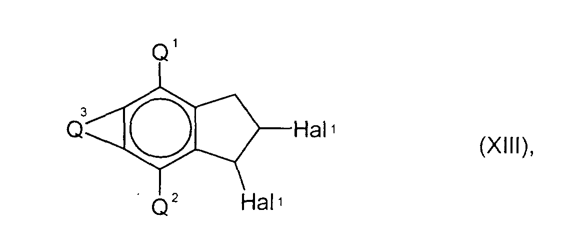 und nachfolgend eine Halogenwasserstoffabspaltung vornimmt. Methoden zur Dihalogenierung und anschließenden Halogenwasserstoffabspaltung sind dem Fachmann allgemein bekannt und beispielsweise in Patai, The Chemistry of Halides, Pseudo-Halides and Azides, S. 1173-1227, New York, Wiley 1983 beschrieben.

Die Erfindung betrifft weiterhin die Verwendung der Verbindungen gemäß Formel (I) als Katalysatoren sowohl auf einem Katalysatorträger (z.B. Al₂O₃, SiO₂ und andere inerte) als auch ohne Träger zur Polymerisation von Monomeren aus der Gruppe von C₂-C₆-α-Olefinen, C₄-C₆-Diolefinen und Cyclo(di)olefinen oder zur Copolymerisation mehrerer der genannten Monomeren, insbesondere zur Herstellung amorpher, weitgehend ataktischer Polymerer.

Die Erfindung betrifft in bevorzugter Weise das beschriebene Verfahren und die damit herstellbaren Verbindungen der Formel (I), worin Y die Bedeutung -Si(R¹R²)-, -Ge(R¹R²)- oder -Si(R¹R²)-Si(R³R⁴)-, besonders bevorzugt -Si(R¹R²)- hat und die Umsetzung von (II) mit (i) Mg bzw. Zn und (ii) mit (III) zum Reaktionsprodukt (IV) simultan erfolgt.

Cyclische π-Systeme im Rahmen der Bedeutung von Z sind beispielsweise substituiertes oder nicht substituiertes Cyclopentadien, substituiertes oder nicht substituiertes 1-Inden, substituiertes oder nicht substituiertes 2-Inden, substituiertes oder nicht substituiertes Fluoren, die mit der Brücke Y kovalent und mit M¹ ionisch, kovalent oder koordinativ gebunden sind.

Die Erfindung betrifft in bevorzugter Weise das erfindungsgemäße Verfahren und erfindungsgemäße metallorganische Verbindungen von Übergangsmetallen der Formel (I), in der jedoch an die Stelle von Z der zweite Ligand Z' tritt, der die Bedeutung substituiertes oder nicht substituiertes Cyclopentadien, substituiertes oder nicht substituiertes 1-Inden, substituiertes oder nicht substituiertes 2-Inden, substituiertes oder nicht substituiertes Fluoren, -N(R⁵)-, -P(R⁶)-, | N(R⁵R⁷)-, l P(R⁶R⁸)-, -O-, -S-, | OR⁵- oder SR⁵- hat, worin R⁵ bis R⁸ und die senkrechten Striche die obengenannte Bedeutung haben.

Weiter bevorzugte zweite Liganden sind solche mit der Formel Z" mit dem Bedeutungsumfang von -N(R⁵)- oder | N(R⁵R⁷)-, insbesondere in Verbindung mit Y = -Si(R¹R²)- und M¹ = Ti oder Zr.

Verbindungen der Formel (I), in denen Y = -Si(R¹R²)-, M¹ = Ti oder Zr und Z = -N(R⁵)- bedeuten, eignen sich insbesondere zur Herstellung von ataktischem Polypropylen.

Geradkettiges oder verzweigtes C₁-C₁₀-Alkyl ist beispielsweise Methyl, Ethyl, Propyl, Isopropyl, Butyl, Isobutyl, tert.-Butyl, die isomeren Pentyle, Hexyle, Octyle oder Decyle. Bevorzugt ist C₁-C₄-Alkyl, besonders bevorzugt sind Methyl und Ethyl.

C₅-C₈-Cycloalkyl ist beispielsweise Cyclopentyl, Methyl-cyclopentyl, Dimethylcyclopentyl, Cyclohexyl, Methyl-cyclohexyl, Dimethyl-cyclohexyl, Cycloheptyl, Cyclooctyl, bevorzugt Cyclopentyl und Cyclohexyl und ihre Methyl- und Dimethyl-Derivate.

C₆-C₁₄-Aryl ist beispielsweise Phenyl, Naphthyl, Biphenylyl, Anthryl, Phenanthryl, bevorzugt Phenyl.

C₇-C₁₀-Aralkyl ist beispielsweise Benzyl, α- oder β-Phenyl-ethyl, Phenyl-propyl oder Phenyl-butyl.

C₁-C₄-Alkoxy bzw. C₁-C₄-Alkylthio sind beispielsweise Methoxy, Methylthio, Ethoxy, Ethylthio, Propoxy, Propylthio, Isopropoxy, Isopropylthio, Butoxy, Butylthio, Isobutoxy und Isobutylthio.

Aryl bzw. die aromatischen Anteile von Aralkyl können 1- oder 2-fach, gleich oder unterschiedlich durch Fluor, Chlor, Brom, Methyl, Ethyl, Methoxy oder Ethoxy substituiert sein.

Q³ ist beispielsweise -(CR¹¹₂)ₘ- , für m = 2, 3, 4, 5 oder 6, wobei R¹¹ den Bedeutungsumfang von R¹ bis R⁴ hat, besonders bevorzugt -(CH₂)ₘ für m = 3, 4.

Halogen im Rahmen von R¹ bis R⁸ ist beispielsweise Fluor, Chlor, Brom oder verschiedene von ihnen, bevorzugt Chlor.

M¹ ist beispielsweise Ti, Zr, Hf, V, Nb, Ta, Cr, W, Mo, bevorzugt Ti, Zr, Hf, V, Nb, besonders bevorzugt Ti, Zr, Hf, ganz besonders bevorzugt Ti, Zr. M¹ kann sowohl in der höchstmöglichen Oxidationsstufe als auch in einer davon abweichenden niedrigeren Oxidationsstufe eingesetzt werden und so in den metallorganischen Verbindungen auftreten. In vielen Fällen ist es vorteilhaft, M¹ zunächst in einer niederen Oxidationsstufe einzusetzen und danach mit einem milden Oxidationsmittel, beispielsweise PbCl₂, aufzuoxidieren.

X ist ein einfach oder mehrfach geladenes Anion aus der Gruppe von Fluorid, Chlorid, Bromid, C₁-C₄-Carboxylat, Amid, C₁-C₄-Alkyl, Phenyl, Benzyl, Neopentyl und substituiertes oder nicht substituiertes Butadienyl, bevorzugt Chlorid oder Fluorid, es können auch verschiedene der genannten Anionen vorliegen.

Hall, Hal² und Hal³ im Rahmen von (II) und (III) sind unabhängig voneinander Cl, Br oder I, bevorzugt sind Hal¹ Br und Hal² und Hal³ Cl oder Br.

Die Temperatur zur Umsetzung von (II) mit Mg oder Zn liegt im Bereich von -20°C bis +120°C, bevorzugt 0°C bis +100°C, besonders bevorzugt +25°C bis +80°C.

Die Menge Mg bzw. Zn beträgt 1 bis 100 Mol pro Mol (II). Grundsätzlich kann auch mit Mengen außerhalb des genannten Bereiches gearbeitet werden. Unterhalb von 1 Mol Mg bzw. Zn pro Mol (II) ist die Umsetzung von (II) unvollständig und oberhalb von 100 Mol ist kein weiterer Vorteil bezüglich Vollständigkeit und Geschwindigkeit der Umsetzung zu erwarten. In bevorzugter Weise werden 1 bis 10 Mol Mg bzw. Zn, in besonders bevorzugter Weise 1 bis 5 Mol Mg bzw. Zn pro Mol (II) eingesetzt. Von den Metallen Mg und Zn ist Mg zur Umsetzung bevorzugt.

Die Temperatur zur weiteren Umsetzung mit (III) liegt ebenfalls im Bereich von - 20°C bis +120°C, bevorzugt 0°C bis +100°C, besonders bevorzugt +25°C bis +80°C.

Die Menge von (III) beträgt 1 bis 20 Mol pro Mol (II). In Mengen außerhalb dieses Bereiches gilt das oben zur Menge an Mg bzw. Zn Gesagte. In bevorzugter Weise werden 1 bis 10 Mol (III), in besonders bevorzugter Weise 1 bis 2 Mol (III) pro Mol (II) eingesetzt.

Nicht umgesetztes Mg bzw. Zn und (III) werden auf fachmännisch bekannte Weise vom Reaktionsansatz abgetrennt und können erneut eingesetzt werden.

Das erfindungsgemäße Verfahren kann in Gegenwart eines polaren, aprotischen Lösungsmittels durchgeführt werden. Geeignete Lösungsmittels sind beispielsweise Methylenchlorid, Chloroform, Dimethylformamid, N-Methyl-pyrrolidon und Ether. Hiervon sind die Ether bevorzugt, beispielsweise Diethylether, Diisopropylether, Dioxan, Tetrahydrofuran und andere dem Fachmann bekannte. Die Menge Lösungsmittel wird so gewählt, dass (II) und die daraus entstehende Mg-organische bzw. Zn-organische Verbindung gelöst vorliegen und das nicht umgesetzte Mg bzw. Zn etwa durch Filtration oder Dekantieren oder analoge Trennoperation abgetrennt werden kann. Diese Menge beträgt beispielsweise 50 bis 1000 % der Menge von (II).

Y ist in bevorzugter Weise -C(R¹R²)-, -Si(R¹R²)-, besonders bevorzugt -Si(R¹R²)-.

Für den Fall, dass Y die Bedeutung -Si(R¹R²)-, -Ge(R¹R²)- oder -Si(R¹R²)-Si(R³R⁴)- hat, eröffnet die simultane Umsetzung von (II) mit (i) Mg bzw. Zn und (ii) mit (III) eine elegante Möglichkeit der Einsparung eines Reaktionsschrittes.

Für den Fall, dass die Umsetzung von (IV) mit (Va) oder (Vb) zu (VII) in Gegenwart einer Hilfsbase durchgeführt wird, kommen hierfür beispielsweise in Betracht: Offenkettige oder cyclische tertiäre aliphatische Amine mit insgesamt 3 bis 30 C-Atomen, wie Trimethylamin, Triethylamin, Tripropylamin, Triisopropylamin, Tributylamin, Triisobutylamin, Trihexylamin, Trioctylamin, Tridecylamin, N-Methyl-piperidin, N,N'-Dimethyl-piperazin, Diaza-bicyclo-nonan (DBN), Diazabicyclo-undecan (DBU), auch Amine mit unterschiedlich langen C-Ketten, wie N,N-Dimethyl-butylamin, N,N-Dimethyl-octylamin, N,N-Dimethyl-stearylamin und ähnliche, und aromatische Amine, wie Pyridin, Methylpyridine, Chinolin, N,N-Dimethyl-anilin und ähnliche.

Die Aufarbeitung des die metallorganische Verbindung (I) enthaltenden Reaktionsgemisches erfolgt mit fachmännisch bekannten Operationen, wie Filtration, Abdestillieren flüchtiger Gemischanteile und Kristallisation.

Die metallorganischen Verbindungen der Formel (I) können als Katalysatoren zur (Co)Polymerisation von C₂-C₁₂-α-Olefinen, C₄-C₂₀-Diolefinen, Cyclo(di)olefinen oder Gemischen mehrerer von ihnen verwendet werden. Monomere der genannten Gruppen sind beispielsweise: Ethylen, Propylen, 1-Butylen, 1-Penten, 1-Hexen, 1-Octen und deren verzweigte Isomere, Isobutylen, 1,3-Butadien, 1,3- oder 1,4-Pentadien, 1,3-, 1,4- oder 1,5-Hexadien, 1,5-Heptadien, Isopren, Chloropren, Norbornen, 5-Ethyliden-2-norbomen, 5-Vinyl-2-norbornen, 4-Vinyl-1-cyclohexen, Dicyclopentadien, 7-Methyl-1,6-octadien und 5,7-Dimethyl-1,6-octadien.

Die Verbindungen der Formel (I) werden zur (Co)Polymerisation häufig in Kombination mit Cokatalysatoren eingesetzt.

Als Cokatalysatoren kommen die auf dem Gebiet der Metallocene bekannten Cokatalysatoren in Frage, wie polymere oder oligomere Alumoxane, Lewissäuren sowie Aluminate und Borate. In diesem Zusammenhang wird insbesondere verwiesen auf Macromol. Symp. Vol. 97, Juli 1995, S. 1 - 246 (für Alumoxane), sowie auf EP-A1-277 003, EP-A1-277 004, Organometallics 1997, 16, 842-857 (für Borate) und EP-A2-573 403 (für Aluminate).

Insbesondere eignen sich als Cokatalysatoren Methylalumoxan, durch Triisobutylaluminium (TIBA) modifiziertes Methylalumoxan, sowie Diisobutylalumoxan, Trialkylaluminiumverbindungen, wie Trimethylaluminium, Triethylaluminium, Triisobutylaluminium, Triisooctylaluminium, darüber hinaus Dialkylaluminium-Verbindungen wie Diisobutylaluminiumhydrid, Diethylaluminiumchlorid, substituierte Triarylborverbindungen, wie Tris(pentafluorphenyl)boran, sowie ionische Verbindungen, die als Anion Tetrakis(pentafluorphenyl)borat enthalten, wie Triphenylmethyltetrakis(pentafluorphenyl)borat, Trimethylammoniumtetrakis-(pentafluorphenyl)borat, N,N-Dimethylaniliniumtetrakis(pentafluorphenyl)borat, substituierte Triarylaluminiumverbindungen, wie Tris(pentafluorphenyl)aluminium, sowie ionische Verbindungen, die als Anion Tetrakis(pentafluor-phenyl)aluminat enthalten, wie Triphenylmethyltetrakis(pentafluorphenyl)aluminat, N,N-Dimethylaniliniumtetrakis(pentafluorphenyl)aluminat.

Selbstverständlich ist es möglich, die Cokatalysatoren im Gemisch untereinander einzusetzen. Die jeweils günstigsten Mischungsverhältnisse sind durch geeignete Vorversuche zu bestimmen.

Solche (Co)Polymerisationen werden in der Gas-, Flüssig- oder Slurryphase ausgeführt. Der Temperaturbereich hierzu reicht von -20°C bis +200°C, bevorzugt 0°C bis 160°C, besonders bevorzugt +20°C bis +80°C; der Druckbereich reicht von 1 bis 50 bar, bevorzugt 3 bis 30 bar. Mitverwendete Lösungsmittel sind beispielsweise: gesättigte Aliphaten oder (Halogen)Aromaten, wie Pentan, Hexan, Heptan, Cyclohexan, Petrolether, Petroleum, hydrierte Benzine, Benzol, Toluol, Xylol, Ethylbenzol, Chlorbenzol und analoge. Diese Reaktionsbedingungen zur (Co)Polymerisation sind dem Fachmann grundsätzlich bekannt.

Wichtige Polymere, die mit den erfindungsgemäßen metallorganischen Verbindungen als Katalysatoren hergestellt werden können, sind solche des Ethylens und Copolymere hiervon. Als Comonomere geeignet sind C₂- bis C₁₂-Alkene, wie Ethylen, 1-Buten, 1-Penten, 1-Hexen, 1-Octen und Arylalkene, wie z.B. Styrol. Ebenfalls geeignete Comonomere sind nichtkonjugierte Diene, wie 1,4-Hexadien, 1,5-Heptadien, 4-Vinyl-1-cyclohexen, 7-Methyl-1,6-octadien und 5,7-Dimethyl-1,6-octadien, 5-Ethyliden-2-norbornen, 5-Vinyl-2-norbornen und Dicyclopentadien. Es ist möglich auch Gemische der genannten Comonomere einzusetzten.

Die so herstellbaren Ethylen(co)polymere besitzen Molekulargewichte mit M_{w} >100 000 g/mol und Molgewichtsverteilungen mit M_{w}/Mₙ <4. Die Ethylen(co)polymere haben intrinsische Viskositäten größer 1 dl/g, bevorzugt größer 2 dl/g. Die Kristallinitäten sind kleiner als 15 %, wobei % Kristallinität = (Schmelzenthalpie/209 J/g) x 100 und die Schmelzenthalpie in J/g mit der DSC-Methode ermittelt wird. Besonders bevorzugt sind Ethylen(co)polymere mit Schmelzenthalpien mit einem Wert von kleiner 5 J/g (DSC-Methode). Die Ethylen(co)polymeren sind gut löslich in gängigen Lösungsmitteln, wie Hexan, Heptan, Diethylether oder Toluol.

In der beschriebenen Weise lassen sich insbesondere auch Kautschuke auf Basis von Ethylen und einem oder mehreren der genannten Comonomere herstellen. Besonders bevorzugt ist die Copolymerisation von Ethylen und Propylen, wobei amorphe Ethylen(co)polymere mit einem Ethylenanteil im Polymeren im Bereich von 30 bis 70 Gew.-%, bevorzugt von 40 bis 65 Gew.-%, erhalten werden.

In der beschriebenen Weise lassen sich auch EPDM-Kautschuke auf Basis von Ethylen, Propylen und einem Dien, vorzugsweise 5-Ethyliden-2-norbomen herstellen. Die EPDM-Kautschuke sind dadurch charakterisiert, dass sie hohe Molekulargewichte und geringe kristalline Anteile aufweisen.

Mit den erfindungsgemäßen metallorganischen Verbindungen lassen sich besonders gut hochmolekulare ataktische Polymere, z. B. ataktisches Polypropylen herstellen.

Beispielsweise kann die (Co)polymerisation von Ethylen mit oder ohne den genannten Comonomeren wie folgt durchgeführt werden: ein Stahlautoklav wird nach den üblichen Reinigungsoperationen mit einem Lösungsmittel und einem Scavenger, z.B. Triisobutylaluminium befüllt. Durch den Scavenger werden mögliche Verunreinigungen und Katalysatorgifte, z.B. Wasser oder andere sauerstoffhaltigen Verbindungen unschädlich gemacht. Dann wird als Katalysatorvorstufe eine Verbindung der Formel (I) zugegeben. Anschließend wird der Reaktor mit Monomeren bis zu einem bestimmten Druck befüllt, auf eine ausgewählte Temperatur thermostatisiert und die Polymerisation durch Zugabe eines oder mehrerer der zuvor genannten Cokatalysatoren gestartet. Die Polymerisation kann in einem kontinuierlichen oder diskontinuierlichen Prozeß erfolgen.

### Beispiele

Die Erfindung wird anhand der nachstehenden Beispiele näher erläutert.

Allgemeine Angaben: Herstellung und Handhabung organometallischer Verbindungen erfolgten unter Ausschluß von Luft und Feuchtigkeit unter Argon-Schutz (Schlenk-Technik). Alle benötigten Lösungsmittel wurden vor Gebrauch durch mehrstündiges Sieden über einem geeigneten Trockenmittel und anschließende Destillation unter Argon absolutiert. Die Verbindungen wurden mit ¹H-NMR, ¹³C-NMR und Infrarotspektroskopie charakterisiert.

### Polymercharakterisierung:

Die intrinsische Viskosität wurde in einem Ubbelohde-Kapillarviskosimeter bei 140°C in o-Dichlorbenzol als Lösungsmittel bestimmt (Mehrpunktmessung). Die DSC-Messungen erfolgten an einem Gerät der Firma Perkin-Elmer mit der Bezeichnung Differential-Scanning-Calorimeter DSC-2 nach folgender Vorschrift: zwei Aufheizungen -90°C, bis +180°C, Heizrate 20K/min, schnelle Abkühlung mit 320K/min auf -90°C, Stickstoffspülung, Einwaagen 12,3 mg Probenmasse in Normkapseln. Die NMR-Messungen zur Bestimmung der Mikrostruktur erfolgten in Tetrachlorethan an einem Gerät der Firma Bruker DRX-400. Die Bestimmung der Mooneyviskosität erfolgte nach ASTM 1646 / DIN 53 523. Die IR-spektroskopische Ermittlung der Polymerzusammensetzung erfolgte gemäß ASTM D 3900.

Abkürzungen:
- d. Th.: der Theorie
- bez. auf: bezogen auf
- TIBA: Triisobutylaluminium
- I.V.: intrinsische Viskosität
- Tg: Glasübergangstemperatur

### Beispiel 1

### Herstellung von 5,6,7-Tetrahydroindacen-1-on

Indan (60.0 g, 62.6 ml, 0.5 mol) und Acrylsäurechlorid (45.9 g, 41.0 ml, 0.5 mol) wurden in einem 1 1 Dreihalsrundkolben mit Rückflußkühler in 600 ml wasserfreiem Methylenchlorid gelöst. Man kühlte auf 0°C und gab Alumniumtrichlorid (135.0 g, 1.0 mol) langsam portionsweise zu. Man erwärmte innerhalb von 30 min auf 25°C und rührte weitere 15 h. Danach wurde die Reaktionsmischung 1 h unter Rückfluß gekocht. Nach dem Abkühlen wurde die Reaktionsmischung in einem 2 1 Becherglas auf ca. 1000 g Eis gegossen. Nach 15 h Stehen wurde die organische Phase abgetrennt und die wässrige Phase mit 100 ml CH₂Cl₂ einmal gewaschen. Die vereinigten organischen Phasen wurden mit wasserfreiem Na₂SO₄ getrocknet und die flüchtigen Bestandteile am Rotationsverdampfer entfernt. Zur Vorreinigung wurde das erhaltene braune Öl durch Kieselgel mit Methylenchlorid filtriert (in zwei Portionen, Säulendimension: 4 x 20 cm, Methylenchlorid, 25°C). Nach Entfernen des Lösungsmittels destillierte man das braune Rohprodukt in einer Mikrodestillationsapparatur ohne Kühlung im Ölpumpenvakuum. Man erhielt einen hellgelben Feststoff, der bei 115 bis 125°C bei 2 mbar (Ölbad: 170 bis 190°C ) überdestillierte.
- Ausbeute:: 21.0 g 5,6,7-Tetrahydroindacen-1-on (0.12 mol, 24 % d.Th., bez. auf eingesetztes Indan)
**IR (KBr) [cm**^{**-1**}**]:** 3039 (s); 2953 (s); 2918 (s); 2841 (s); ν_{CO} =1692 (bs); 1611 (s); 1573 (s); 1435 (s); 1304 (s); 1247 (s).
^{**1**}**H NMR (CDCl**_{**3**}**):** δ 7.54 (s, 1 H, C_{arom.}-H); 7.27 (s, 1 H, C_{arom.}-H); 3.05 (t, 2 H, ³J_{HH} = 6.0 Hz, CO-CH₂), 2.93 (t, 2 H, ³J_{HH} = 8.0 Hz, CH₂-CH₂-CH=), 2.90 (t, 2 H, ³J_{HH} = 8.0 Hz, CH₂-CH₂-CH=), 2.66 (t, 2 H, ³J_{HH} = 6.0 Hz, CO-CH₂-CH₂), 2.10 (pq, 2 H, ³J_{HH} = 8.0 Hz, CH₂-CH₂-CH=).
^{**13**}**C NMR (CDCl**_{**3**}**):** δ 206.5 (C=O), 154.3 (C-CH₂-CH₂), 152.8 (C-CH₂-CH₂), 144.0 (C-CH₂-CO), 135.0 (C-CH₂-CH₂-CO), 122.0 (CH, C_{arom.}), 118.8 (CH, C_{arom.}), 36.7 (CH₂), 33.0 (CH₂), 31.9 (CH2), 25.8 (CH₂), 25.5 (CH₂).

### Beispiel 2

### Herstellung von 5,6,7-Tetrahydroindacen-1-ol

5,6,7-Tetrahydroindacen-1-on (20.0 g, 0.125 mol) wurden in 100 ml absolutem Diethylether gelöst. Bei 0°C wurde fein gepulvertes NaBH4 (4.06 g, 0.125 mol) zugegeben. Nun wurden 50 ml absolutes Ethanol langsam zugetropft. Nach 15 h rühren bei 25°C (H₂-Entwichlung !) wurde die Suspension auf Eis gegeben. Es bildete sich ein farbloser Feststoff. Nach Ansäuern mit 150 ml 1 M HCl und Zugabe von weiteren 50 ml Diethylether wurde die organische Phase zweimal mit je 25 ml 1 molarer HCl gewaschen und die HCl-haltige Phase abgetrennt. Nach Entfernen aller flüchtigen Bestandteile am Rotationsverdampfer, erhielt man einen farblosen Feststoff von 5,6,7-Tetrahydroindacen-1-ol, der ohne weitere Reinigung in Beispiel 3 eingesetzt wurde.

### Beispiel 3

### Herstellung von 5,6,7-Tetrahydroindacen

Dazu wurde das in Beispiel 2 hergestellte 5,6,7-Tetrahydroindacen-1-ol in 200 ml Benzol gelöst und mit 300 mg p-Toluolsulfonsäure versetzt. Man kochte unter Rückfluß am Wasserabscheider (4 bis 5 h), wobei das während der Reaktion gebildete Wasser entfernt wurde. Nun wurde die Reaktionslösung zweimal mit je 25 ml 1 molarer NaHCO₃ gewaschen, dann das Benzol am Rotationsverdampfer entfernt. Der erhaltene Rückstand wurde säulenchromatographisch (Säulendimension: 4 x 20 cm, Kieselgel, Petrolether, 25°C) gereinigt. Man erhielt bei 25°C ein farbloses Öl, welches im Kühlschrank erstarrte.
- Ausbeute:: 16.3 g 5,6,7-Tetrahydroindacen (0.113 mol, 90 % d. Th. bez. auf eingesetztes Keton)
**IR (NaCl) [cm**^{**-1**}**]:** 3059 (m), 2997 (m), 2952 (m), 2881 (m), 2838 (m), 1853 (m), 1732 (breit, m), 1616 (s), 1543 (s), 1455 (breit, s), 938 (breit, s), 861 (breit, s), 813 (breit, s).
^{**1**}**H NMR (CDCl**_{**3**}**): δ** 7.41 (s, 1 H, C_{arom.}-H), 7.38 (s, 1 H, C_{arom.}-H), 6.92 (d, 1 H, ³J_{HH} = 5.0 Hz, C-CH=CH-CH2), 6.57 (d, 1 H, ³J_{HH} = 5.0 Hz, C-CH=CH-CH2), 3.43 (s, 2 H, C-CH=CH-CH2), 3.00 (pt, 4 H, ³J_{HH} = 7.5 Hz, CH₂-CH₂-CH=), 2.19 (pq, 2 H, ³J_{HH} = 7.5 Hz, CH₂-CH₂-CH=).
^{**13**}**C NMR (CDCl**_{**3**}**): δ** 143.3 (C_{arom.}), 142.2 (C_{arom.}), 142.1 (C_{arom.}), 140.8 (C_{arom.}), 133.3 (C-CH=CH-CH₂), 131.9 (C-CH=CH-CH₂), 119.8 (C_{arom.}-H), 116.8 (C_{arom.}-H), 38.5 (CH₂), 32.65 (CH₂), 32.60 (CH₂), 25.9 (CH₂).

### Beispiel 4

### Herstellung von 1,2-Dibrom-5,6,7-tetrahydroindacan

5,6,7-Tetrahydroindacen (7.6 g, 0.0486 mol) aus Beispiel 3 wurde in 100 ml Diethylether gelöst. Bei 0°C wurde Brom (7.8 g, 2.5 ml, 0.0486 mol) langsam zugetropft. Es wurde 1 h bei 0°C und anschließend 12 h bei 25°C gerührt. Der Diethylether wurde am Rotationverdampfer entfernt und der Rückstand an Kieselgel chromatographiert (Säulendimension: 4 x 20 cm, Methylenchlorid/Hexan = 1:10, 25°C). Man erhielt 1,2-Dibrom-5,6,7-tetrahydroindacan als ein hellgelbes Öl.
- Ausbeute:: 11.8 g 1,2-Dibrom-5,6,7-tetrahydroindacan (0.0373 mol, 77 % d. Th. bez. auf eingesetztes 5,6,7-Tetrahydroindacen).
**IR (NaCl) [cm**^{**-1**}**]:** 3011 (s), 2947 (breit, s), 2893 (s), 2851 (s), 1746 (w), 1618 (w), 1436 (breit, s), 1315 (s), 1284 (s), 1254 (s), 1208 (s), 1145 (s), 945 (w), 915 (m).
^{**1**}**H NMR (CDCl**_{**3**}**): δ** 7.51 (s, 1 H, Cₐᵣₒₘ-H), 7.25 (s, 1 H, Cₐᵣₒₘ-H), 6.51 (s, 1 H, C_{arom.}-CHBr-CHBr)¹, 4.74 (d, 1 H, ³J_{HH} = 4.0 Hz, C_{arom.}-CHBr-CHBr), 3.62 (dd, 1 H, ³J_{HH} = 4.0, 12.0 Hz, C_{arom.}-CH₂-CHBr), 3.05 (d, 1 H, ³J_{HH} = 12.0 Hz, C_{arom.}-CH₂-CHBr).
^{**13**}**C NMR (CDCl**_{**3**}**): δ** 147.2 (C_{arom.}), 144.9 (C_{arom.}), 139.4 (C_{arom.}), 138.9 (C_{arom.}), 122.0 (C_{arom.}-H), 121.8 (C_{arom.}-H), 59.2 (CHBr), 55.5 (CHBr), 41.6(CH₂), 33.5 (CH₂), 33.3 (CH₂), 26.2(CH2).

### Beispiel 5

### Herstellung von 2-Brom-5,6,7-tetrahydroindacen

1,2-Dibrom-5,6,7-tetrahydroindacan (11.9 g, 0.0373 mol) aus Beispiel 4 wurde in 50 ml Tetralin gelöst und zur Bromwasserstoffeliminierung 4 h unter Rückfluß erhitzt. Das Tetralin wurde danach destillativ im Ölpumpenvakuum entfernt. Zur vollständigen Entfernung des Tetralins wurde 10 min auf 100°C im Ölpumpenvakuum erhitzt.

Zur weiteren Reinigung wurde der Rückstand durch Kieselgel chromatographiert. Als Eluent diente eine Mischung aus Methylenchlorid und Hexan im Verhältnis 1:3. Das so erhaltene Rohprodukt wurde durch Kristallisation aus Methanol gereinigt. Man erhielt farblose Kristalle von 2-Brom-5,6,7-tetrahydroindacen.
- Ausbeute:: 1.2 g 2-Brom-5,6,7-tetrahydroindacen (0.0051 mol, 14 % d. Th., bez. auf eingesetztes 1 ,2-Dibrom-5,6,7-tetrahydroindacan).
**Schmp.:** 95°C.
**IR (KBr) [cm**^{**-1**}**]:** 3013 (w), 2944 (s), 2842 (s), 1544 (m), 1459 (s), 1391 (s), 1255 (s), 877 (s).
^{**1**}**H NMR (CDCl**_{**3**}**): δ** 7.24 (s, 1 H, C_{arom.}-H), 7.18 (s, 1 H, C_{arom.}-H), 6.78 (s, 1 H, C_{arom.}-CH=CBr), 3.55 (s, 2 H, CH=CBr-CH2), 2.93 (pt, 4 H, ³J_{HH} = 7.5 Hz, CH₂-CH₂-CH=), 2.12 (pq, 4 H, ³J_{HH} = 7.5 Hz, CH₂-CH₂-CH=).
^{**13**}**C NMR (CDCl**_{**3**}**): δ** 143.0 (C_{arom.}), 142.7 (C_{arom.}), 141.5 (C_{arom.}), 141.3 (C_{arom.}), 133.6 (CH=CBr), 123.8 (CBr), 119.9 (C_{arom.}-H), 116.6 (C_{arom.}-H), 45.4 (CBr-CH₂), 33.1(CH₂-CH₂-Cₐᵣₒₘ), 33.1(CH₂-CH₂-Cₐᵣₒₘ), 26.1(CH₂-CH₂-Cₐᵣₒₘ).

### Beispiel 6

### Herstellung von [5,6,7-Tetrahydroindacenyl]dimethylchlorsilan

2-Brom-5,6,7-tetrahydroindacen (1.2 g, 0.0051 mol) aus Beispiel 5 wurde in 4.0 ml Tetrahydrofuran gelöst und zu einer Mischung bestehend aus Magnesium (0.2 g, 0.008 mol), Dichlordimethylsilan (1.5 g, 1.3 ml, 0.012 mol) in 2 ml Tetrahydrofuran langsam addiert. Dabei erwärmte sich die Reaktionsmischung auf 60°C. Nach Rühren bei 25°C für 15 h werden alle flüchtigen Bestandteile im Ölpumpenvakuum entfernt und der Rückstand in 50 ml Petrolether aufgenommen. Die ausgefallenen Magnesiumsalze wurden durch Filtration (Fritte) entfernt und das Filtrat im Ölpumpenvakuum vom Lösungsmittel befreit. Man erhielt einen hellgelben wachsartigen Feststoff, der ohne weitere Reinigung zur Synthese von ^{t}Butylamin-2-[5,6,7-tetrahydroindacenyl]dimethylsilan (Beispiel 7) eingesetzt wurde.
- Ausb.:: 1.23 g [5,6,7-Tetrahydroindacenyl]dimethylchlorsilan (4.94 mmol, 97 % d. Th., bez. auf eingesetztes 2-Brom-5,6,7-tetrahydroindacen).

### Beispiel 7

### Herstellung von ^{t}Butylamin-2-[5,6,7-tetrahydroindacenyl]dimethylsilan

Dazu wurde das in Beispiel 6 erhaltene [5,6,7-Tetrahydroindacenyl]dimethylchlorsilan in 20.0 ml Diethylether gelöst, auf 0°C gekühlt und mit ^{t}Butylamin (2.0 ml, 0.025 mol) in einer Portion versetzt. Es wurde 15 h bei 25°C gerührt. Dann wurden alle flüchtigen Bestandteile entfernt. Der Rückstand wurde in 40 ml Petrolether aufgenommen und das ausgefallene Ammoniumsalz durch Filtration abgetrennt. Das Filtrat wurde von allen flüchtigen Bestandteilen im Ölpumpenvakuum befreit. Man erhielt ein hellgelbes Öl von ^{t}Butylamin-2-[5,6,7-tetrahydroindacenyl]dimethylsilan.
- Ausbeute:: 1.32 g ^{t}Butylamin-2-[5,6,7-tetrahydroindacenyl]dimethylsilan (4.6 mmol, 94 % d. Th., bez. auf eingesetztes 2-Brom-5,6,7-tetrahydroindacen).
**IR (NaCl) [cm**^{**-1**}**]:** 3382 (m), 3053 (w), 3004 (m), 2959 (s), 2893 (s), 2844 (m), 1533 (m), 1462 (m), 1376 (m), 1251 (s), 1225 (s), 1090 (breit, m), 1035 (breit, m), 849 (breit, s).
^{**1**}**H NMR (CDCl**_{**3**}**):** δ 7.22 (s, 1 H, C_{arom.}-H), 7.14 (s, 1 H, C_{arom.}-H), 7.01 (s, 1 H, C_{arom.}-CH=CSi), 3.42 (s, 2 H, CH=CSi-CH₂), 2.93 (pt, 4 H, ³J_{HH} = 7.0 Hz, CH₂-CH₂-CH=), 2.01 (pq, 4 H, ³J_{HH} = 7.0 Hz, CH₂-CH₂-CH=), 1.05 (s, 9 H, C(CH3)3), 0.20 (s, 6 H, Si(CH3)2).
^{**13**}**C NMR (CDCl**_{**3**}**):** δ 148.6 (C_{arom.}-Si), 144.3 (C_{arom.}), 143.3 (C_{arom.}), 141.1 (C_{arom.}), 140.0 (C_{arom.}), 139.7 (CH=CSi), 118.5 (C_{arom.}-H), 115.4 (C_{arom.}-H), 48.3 (C(CH3)3), 40.5 (CH-CSi-CH₂), 32.6 (C(CH₃)₃), 31.5 (CH₂-CH₂-Cₐᵣₒₘ), 31.4 (CH₂-CH₂-Cₐᵣₒₘ), 24.9 (CH₂-CH₂-Cₐᵣₒₘ), 0.0 (Si(CH₃)₂).

### Beispiel 8

### Herstellung von ^{t}Butylamin-2-[5,6,7-tetrahydroindacenyl]dimethylsilyl-titaniumdichlorid

^{t}Butylamin-2-[5,6,7-tetrahydroindacenyl]dimethylsilan (0.61 g, 0.00213 mol) aus Beispiel 7 wurde in 15 ml n-Pentan gelöst und bei -78°C mit 1.7 ml einer 2.5 M Lösung von n-BuLi in Hexan tropfenweise versetzt. Man ließ 1 h bei -78°C rühren und dann weitere 2 h bei 25°C. Das Lösungsmittel wurde im Ölpumpenvakuum entfernt, das zurückgebliebene hellorangene Pulver bei -78°C in 20 ml Tetrahydrofuran gelöst und zu einer Suspension von TiCl₃ 3THF in 10 ml Tetrahydrofuran bei -78°C mittels Kanüle transferiert, wobei eine tiefgelbe Färbung eintrat. Nach 1 h Rühren bei -78°C und 1 h bei 25°C wurde festes feingepulvertes Bleidichlorid (0.592 g, 0.00213 mol) zugegeben und 0.5 h bei 25°C gerührt. Die Suspension färbte sich nun rotbraun. Das Lösungsmittel wurde entfernt und der Rückstand zweimal mit je 10 ml Toluol extrahiert. Man ließ die unlöslichen Bestandteile der Suspension sedimentieren und transferierte die darüberstehende Lösung mittels Kanüle in einen Schlenkkolben. Das Toluol wurde entfernt und der Rückstand in 2 ml Petrolether aufgenommen. Bei 25 °C setzte sich ein rotbrauner Feststoff ab.
- Ausbeute:: 0.5 g ^{t}Butylamin-2-[5,6,7-tetrahydroindacenyl]dimethylsilyl-titaniumdichlorid (1.2 mmol, 60 % d. Th., bez. eingesetztes ^{t}Butylamin-2-[5,6,7-tetrahydroindacenyl]dimethylsilan).
**Schmp.:** 153°C.
**IR (KBr) [cm**^{**-1**}**]:** 2957 (s), 2875 (s), 2795 (s), 2691 (m), 2588 (m), 2493 (m), 1606 (m), 1511 (m), 1459 (m), 1433 (m), 1401 (m), 1376 (m), 1300 (w), 1250 (s), 1085 (s), 926 (breit, s), 836 (breit, s), 764 (breit, s).
^{**1**}**H NMR (CDCl**_{**3**}**):** δ 7.40 (s, 2 H, C_{arom.}-H), 6.54 (s, 2 H, C_{arom.}-CH=CSi), 2.92 (pt, 4 H, ³J_{HH} = 7.0 Hz, CH2-CH2-CH=), 2.03 (pq, 2 H, ³J_{HH} = 7.0 Hz, CH₂-CH₂-CH=), 1.30 (s, 9 H, C(CH3)3), 0.67 (s, 6 H, Si(CH₃)₂).
^{**13**}**C NMR (CDCl**_{**3**}**):** δ 147.3 (C_{arom.}), 134.9 (C_{arom.}), 119.2 (C_{arom.}-H), 117.9 (C_{arom.}-H), 113.6 (C_{arom.}-Si), 64.6 (C(CH₃)₃), 32.5 (C(CH₃)₃), 32.3 (CH₂-CH₂-Cₐᵣₒₘ), 25.9 (CH₂-CH₂-Cₐᵣₒₘ) 0.0 (Si(CH₃)₂).

### Beispiel 9

**Polymerisation von Propylen**

In einen 250 ml Glasreaktor wurden 100 ml Toluol, 0.25 ml TIBA und 4,0 mg (10 µmol) ^{t}Butylamin-2-[5,6,7-tetrahydroindacenyl]dimethylsilyl-titaniumdichlorid aus Beispiel 8 bei 20°C vorgelegt. Anschließend wurde mit einem Gaseinleitungsrohr kontinuierlich bei 1,1 bar Druck Propylen in die Lösung eingeleitet. Die Polymerisation wurde durch Zugabe einer Lösung von 18,4 mg (20 µmol) Triphenylmethyl-tetrakis(pentafluorphenyl)borat in 4 ml Toluol gestartet. Die Temperatur stieg während der Polymerisation auf 32°C an. Nach 1 Stunde Polymerisationsdauer wurde eine klare hochviskose Reaktionslösung erhalten. Zur Aufarbeitung wurde die Reaktionslösung in Methanol eingerührt, das gefällte elastische Polymer mit Methanol gewaschen und im Vakuumtrockenschrank getrocknet. Es wurden 13,8 g amorphes hochmolekulares Polypropylen erhalten. Die Messung der intrinsischen Viskosität ergab I.V. = 1,3 dl/g. Nach DSC Messungen betrug die Schmelzenthalpie Null J/g. Die ¹³C-NMR-spektroskopische Untersuchung ergab folgende Zusammensetzung: % mm = 15,6 (isotaktischer Anteil); %(mr/rm) = 51,8 (ataktischer Anteil); % rr = 32,6 (syndiotaktischer Anteil).

### Beispiel 10

### Terpolymerisation von Ethylen, Propylen und 5-Ethyliden-2-norbornen (ENB)

In einen 1,4-1-Stahl-Autoklaven, der mit einem mechanischen Rührer, Manometer, Temperaturfühler, einer Temperatursteuervorrichtung, einer Katalysatorschleuse und Monomerdosiervorrichtungen für Ethylen und Propylen ausgestattet ist, wurden 500 ml Hexan und 1 ml TIBA vorgelegt. Hierzu wurde eine Lösung von 2,0 mg (5 µmol) ^{t}Butylamin-2-[5,6,7-tetrahydroindacenyl]dimethylsilyl-titaniumdichlorid aus Beispiel 8 in 5 ml Toluol gegeben. Die Innentemperatur wurde mit einem Thermostaten auf 40°C eingestellt. Anschließend wurden 16 g Ethylen und 16 g Propylen zudosiert. Durch Zugabe einer Lösung von 9,22 mg (10 µmol) Triphenylmethyltetrakis(pentafluorphenyl)borat in 5 ml Toluol wurde die Polymerisation gestartet. Dann wurden über eine Druckschleuse 5 ml ENB zugegeben. Ethylen und Propylen wurden im Massenverhältnis von 50:50 kontinuierlich zudosiert, so dass der Druck bei 40°C konstant 6 bar betrug. Nach 20 Minuten Polymerisationsdauer wurde erneut eine Lösung von 9,22 mg (10 µmol) Triphenylmethyl-tetrakis(pentafluorphenyl)borat in 5 ml Toluol in den Autoklaven dosiert. Nach insgesamt 60 Minuten Polymerisationsdauer wurde der Autoklav entspannt. Zur Aufarbeitung wurde das Polymer in Methanol ausgefällt, und 20 h bei 60°C im Vakuum getrocknet, wobei 41,0 g Copolymer erhalten wurden. Die IR-spektroskopische Ermittlung der Zusammensetzung des Copolymeren ergab einen Einbau von 47,3 Gew.-% Ethylen, 45,5 Gew.-% Propylen und 7,7 Gew.-% ENB. Entsprechend der DSC-Messung ist das Copolymer vollständig amorph. Die Schmelzenthalpie betrug Null J/g. Mit der DSC-Methode wurde eine Tg von -48°C ermittelt. Die Bestimmung der intrinsischen Viskosität ergab I.V. = 4,1 dl/g.

### Beispiel 11

### Terpolymerisation von Ethylen, Propylen und 5-Ethyliden-2-norbornen (ENB)

Die Polymerisation aus Beispiel 10 wurde wiederholt, mit dem Unterschied, dass 12 g Propylen und 18 g Ethylen in den Autoklaven vorgelegt wurden und Ethylen und Propylen im Massenverhältnis 60:40 kontinuierlich zudosiert wurden. Die Polymerisationsdauer betrug 60 Minuten. Man erhielt 45,1 g eines Terpolymeren mit einem Gehalt von 56,9 Gew.-% Ethylen, 36,8 Gew.-% Propylen und 6,7 Gew.-% ENB (IR-Spektroskopie). Mit der DSC-Methode wurde ein Tg von -47°C ermittelt. Die Messung der intrinsischen Viskosität ergab einen Wert von I.V. = 3,1 dl/g.

### Beispiel 12

### Terpolymerisation von Ethylen, Propylen und 5-Ethyliden-2-norbornen (ENB)

Die Polymerisation aus Beispiel 10 wurde wiederholt, mit dem Unterschied, dass 8 g Propylen und 19 g Ethylen in den Autoklaven vorgelegt wurden und Ethylen und Propylen im Massenverhältnis 70:30 kontinuierlich zudosiert wurden. Die Polymerisationsdauer betrug 60 Minuten. Man erhielt 44,4 g eines Terpolymeren mit einem Gehalt von 64,9 Gew.-% Ethylen, 27,8 Gew.-% Propylen und 7,9 Gew.-% ENB (IR-Spektroskopie). Mit der DSC-Methode wurde ein Tg von -38°C ermittelt. Die Messung der intrinsischen Viskosität ergab einen Wert von I.V. = 3,4 dl/g.

## Patentansprüche

1. Verfahren zur Herstellung von metallorganischen Verbindungen von Übergangsmetallen mit in 5,6-Position anellierten 2-Indenyl als erstem Liganden der Formel 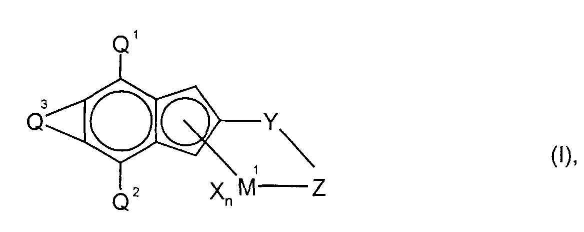 worin
Q¹, Q² gleich oder verschieden sind und als Substituent des in 5,6-Position anellierten 2-Indenylsystems Wasserstoff, C₁-C₄-Alkyl, C₆-C₁₄-Aryl, C₇-C₁₀-Aralkyl, C₁-C₄-Alkoxy, C₁-C₄-Alkylthio, Phenoxy, Phenylthio, Di-C₁-C₄-alkyl-amino, C₆-C₁₄-Aryl-C₁-C₄-alkyl-amino, Di-C₆-C₁₄-aryl-amino, Dibenzylamino, Tri-C₁-C₄-alkyl-silyl, Di-C₁-C₄-alkyl-boranyl, Phenyl-C₁-C₄-alkyl-boranyl, Diphenylboranyl, Di-C₁-C₄-alkyl-phosphoryl, Diphenylphosphoryl oder Phenyl-C₁-C₄-alkylphosphoryl bedeuten,
Q³ einen gegebenfalls substituierten Alkylenrest darstellt, der zusammen mit den beiden Kohlenstoffatomen des Indenylrestes in 5 und 6 Position ein Ringsystem bildet,
M¹ ein Übergangsmetall aus der 4., 5. oder 6. Gruppe des Periodensystems der Elemente nach IUPAC 1985 ist,
X ein Anion bedeutet,
n eine Zahl von Null bis Vier ist, die sich aus der Valenz und dem Bindungszustand von M¹ ergibt,
Y eine Brücke aus der Gruppe von -C(R¹R²)-, -Si(R¹R²)-, -Ge(R¹R²)-, -C(R¹R²)-C(R³R⁴)-, -C(R¹R²)-Si(R³R⁴)- oder -Si(R¹R²)-Si(R³R⁴)-darstellt, worin R¹, R², R³ und R⁴ unabhängig voneinander Wasserstoff, Halogen, geradkettiges oder verzweigtes C₁-C₁₀-Alkyl, C₅-C₈-Cycloalkyl, C₆-C₁₄-Aryl oder C₇-C₁₀-Aralkyl bedeuten, und
Z ein zweiter Ligand aus der Gruppe von offenkettigen und cyclischen, gegebenenfalls anionischen π-Systemen, -N(R⁵)-, -P(R⁶)-, | N(R⁵R⁷)-, | P(R⁶R⁸)-, -O-, -S-, | OR⁵- oder | SR⁵- ist, wobei der waagerechte Strich links vom Elementsymbol N, P, O bzw. S eine kovalente Bindung zwischen Z und M¹ darstellt und wobei der senkrechte Strich links vom Elementsymbol N, P, O bzw. S ein Elektronenpaar bedeutet und die Bindung zwischen Z und M¹ koordinativen (nicht kovalenten) Charakter hat und worin R⁵, R⁶, R⁷ und R⁸ unabhängig voneinander den Bedeutungsumfang von R¹ bis R⁴ haben und R⁵ und R⁷ zusätzlich -Si(R¹R²R³) bedeuten können und R⁶ und R⁸ zusätzlich -Si(R¹R²R³), -OR¹, -SR¹ oder -N(R¹R²) bedeuten können,
dadurch gekennzeichnet, dass man ein in 5,6 Position anelliertes Halogeninden der Formel 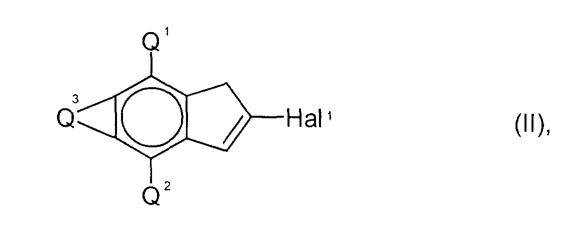 in der Hall für Cl, Br oder I steht und Q¹, Q² und Q³ die obige Bedeutung haben,
mit einem elementaren Metall ausgewählt aus der 1., 2. oder 12. Gruppe des Periodensystems der Elemente nach IUPAC 1985 oder einer entsprechenden Metallverbindung in einer Menge im Bereich von 1 bis 100 Mol Metall/Metallverbindungen pro Mol (II) und mit einem Dihalogenid der Brücke Y der Formel
Hal² - Y - Hal³ (III),
in der Hal² und Hal³ unabhängig voneinander Cl, Br oder I bedeuten und
Y den obigen Bedeutungsumfang hat,
in einer Menge im Bereich von 1 bis 20 Mol (III) pro Mol (II) umsetzt, wobei in dem Fall, dass Y die Bedeutung -Si(R¹R²)-, -Ge(R¹R²)- oder -Si(R¹R²)-Si(R³R⁴)- hat, die Umsetzung von (II) mit (i) Metall/Metallverbindung und (ii) mit (III) auch simultan erfolgen kann, und das Reaktionsprodukt der Formel 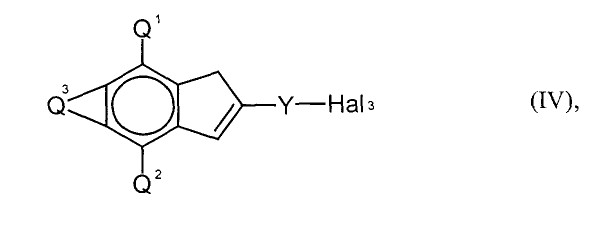 worin Q¹, Q², Q³, Y und Hal³ die obige Bedeutung haben, gegebenenfalls nach seiner Isolierung mit einem Z-Derivat der Formel
ZM² ₚ (Va)
oder
ZR⁹ ₚ (Vb),
in welcher
M² für Li, Na, K oder -MgHal⁴, worin Hal⁴ den Bedeutungsumfang von Hal² hat, steht,
p die Zahl Eins oder Zwei darstellt,
R⁹ Wasserstoff, -Si(R¹R²R³) oder Sn(R¹R²R³) darstellt und
Z, R¹, R² und R³ die obige Bedeutung haben,
gegebenenfalls in Gegenwart einer Hilfsbase zur 2-Indenyl-Verbindung der Formel 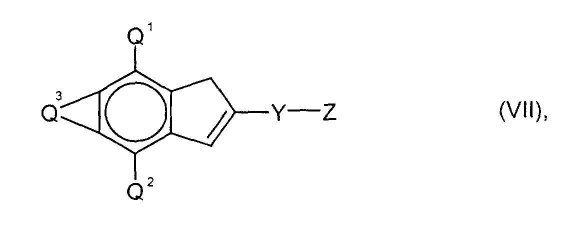 in der Q¹, Q², Q³, Y und Z die obige Bedeutung haben und die als Dianion vorliegen kann und in der Z weiterhin M², R⁹ oder ein Elektronenpaar tragen kann,
und dann weiter mit einer Übergangsmetallverbindung der Formel
M¹X_{q} (VIII),
umsetzt, in der
M¹ und X die obige Bedeutung haben und
q eine Zahl von Zwei bis Sechs ist, die sich aus der Oxidationsstufe von M¹ ergibt.

2. Verfahren nach Anspruch 1, dadurch gekennzeichnet, dass Y eine Brücke aus der Gruppe -Si(R¹R²)-, -Ge(R¹R²)- und -Si(R¹R²)-Si(R³R⁴)-, bevorzugt -Si(R¹R²)- ist und die Umsetzung von (II) mit (i) einer elementaren Metall/Metallverbindung und (ii) mit (III) zum Reaktionsprodukt simultan erfolgt.

3. Verfahren nach Anspruch 1 und/oder 2, dadurch gekennzeichnet, dass man als elementares Metall Mg oder Zn oder ein Gemisch aus Mg und Zn einsetzt.

4. Verfahren nach einem oder mehreren der Ansprüche 1 bis 3, dadurch gekennzeichnet, dass M¹ ein Übergangsmetall aus der Gruppe Ti, Zr, Hf, V, Nb, bevorzugt von Ti, Zr, Hf, besonders bevorzugt von Ti, Zr ist.

5. Verfahren nach einem oder mehreren der Ansprüche 1 bis 4, dadurch gekennzeichnet, dass 1 bis 10 Mol elementarem Metall/Metallverbindung pro Mol (II) und 1 bis 10 Mol (III) pro Mol (II) eingesetzt werden.

6. Metallorganische Verbindungen von Übergangsmetallen mit in 5,6-Position anellierten 2-Indenyl als erstem Liganden der Formel 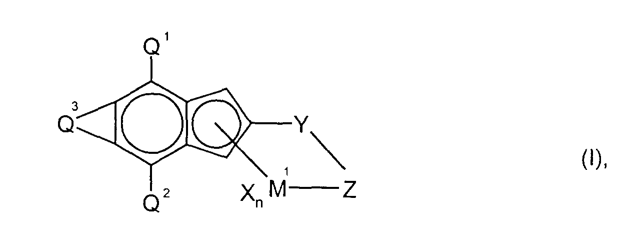 worin
Q¹, Q² gleich oder verschieden sind und als Substituent des in 5,6-Position anellierten 2-Indenylsystems Wasserstoff, C₁-C₄-Alkyl, C₆-C₁₄-Aryl, C₇-C₁₀-Aralkyl, C₁-C₄-Alkoxy, C₁-C₄-Alkylthio, Phenoxy, Phenylthio, Di-C₁-C₄-alkyl-amino, C₆-C₁₄-Aryl-C₁-C₄-alkyl-amino, Di-C₆-C₁₄-aryl-amino, Dibenzylamino, Tri-C₁-C4-alkyl-silyl, Di-C₁-C₄-alkyl-boranyl, Phenyl-C₁-C₄-alkyl-boranyl, Diphenylboranyl, Di-C₁-C₄-alkyl-phosphoryl, Diphenylphosphoryl oder Phenyl-C₁-C₄-alkylphosphoryl bedeuten,
Q³ einen gegebenfalls substituierten Alkylenrest darstellt, der zusammen mit den beiden Kohlenstoffatomen des Indenylrestes in 5 und 6 Position ein Ringsystem bildet,
M¹ ein Übergangsmetall aus der 4., 5. oder 6. Gruppe des Periodensystems der Elemente nach IUPAC 1985 ist,
X ein Anion bedeutet,
n eine Zahl von Null bis Vier ist, die sich aus der Valenz und dem Bindungszustand von M¹ ergibt,
Y eine Brücke aus der Gruppe von -C(R¹R²)-, -Si(R¹R²)-, -Ge(R¹R²)-, -C(R¹R²)-C(R³R⁴)-, -C(R¹R²)-Si(R³R⁴)- oder -Si(R¹R²)-Si(R³R⁴)-darstellt, worin R¹, R², R³ und R⁴ unabhängig voneinander Wasserstoff, Halogen, geradkettiges oder verzweigtes C₁-C₁₀-Alkyl, C₅-C₈-Cycloalkyl, C₆-C₁₄-Aryl oder C₇-C₁₀-Aralkyl bedeuten, und
Z ein zweiter Ligand aus der Gruppe von offenkettigen und cyclischen, gegebenenfalls anionischen π-Systemen, -N(R⁵)-, -P(R⁶)-, |N(R⁵R⁷)-, | P(R⁶R⁸)-, -O-, -S-, | OR⁵- oder | SR⁵- ist, wobei der waagerechte Strich links vom Elementsymbol N, P, O bzw. S eine kovalente Bindung zwischen Z und M¹ darstellt und wobei der senkrechte Strich links vom Elementsymbol N, P, O bzw. S ein Elektronenpaar bedeutet und die Bindung zwischen Z und M¹ koordinativen (nicht kovalenten) Charakter hat und worin R⁵, R⁶, R⁷ und R⁸ unabhängig voneinander den Bedeutungsumfang von R¹ bis R⁴ haben und R⁵ und R⁷ zusätzlich -Si(R¹R²R³) bedeuten können und R⁶ und R⁸ zusätzlich -Si(R¹R²R³), -OR¹, -SR¹ oder -N(R¹R²) bedeuten können.

7. Metallorganische Verbindungen von Übergangsmetallen nach Anspruch 6, worin in Formel (I) an die Stelle von Z der zweite Ligand Z' tritt, der die Bedeutung substituiertes oder nicht substituiertes Cyclopentadienyl, substituiertes oder nicht substituiertes 1-Indenyl, substituiertes oder nicht substituiertes 2-Indenyl, substituiertes oder nicht substituiertes Fluorenyl, -N(R⁵)-, -P(R⁶)-, | N(R⁵R⁷)-, l P(R⁶R⁸)-, -O-, -S-, | OR⁵- oder | SR⁵- hat, worin R⁵ bis R⁸ und die senkrechten Striche die in Anspruch 5 genannte Bedeutung haben.

8. Metallorganische Verbindungen von Übergangsmetallen nach Anspruch 7, worin in Formel (I) an die Stelle von Z' der zweite Ligand Z" tritt, der die Bedeutung -N(R⁵)- oder | N(R⁵R⁷)- hat, wobei bevorzugt in Formel (I) weiterhin Y = -Si(R¹R²)- und M¹ = Ti oder Zr bedeuten, wobei R¹, R², R⁵ und R⁷ sowie der senkrechte Strich neben dem Elementsymbol N die in Anspruch 5 genannte Bedeutung haben.

9. Tert.-Butylamin-2-[5,6,7-tetrahydroindacenyl]dimethylsilyl-titandichlorid.

10. Zwischenprodukte der Formel 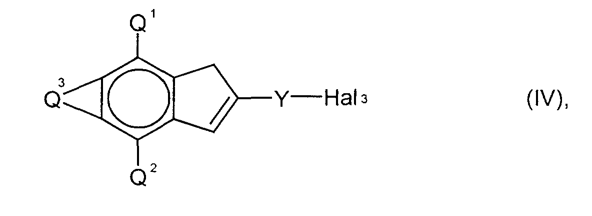 in der
Hall, Q¹, Q² und Q³ die in Anspruch 1 genannte Bedeutung haben.

11. Verfahren zur Herstellung der Zwischenprodukte der Formel (IV) aus Anspruch 10, das dadurch gekennzeichnet ist, dass man ein in 5,6-Position anelliertes 2-Halogeninden der Formel 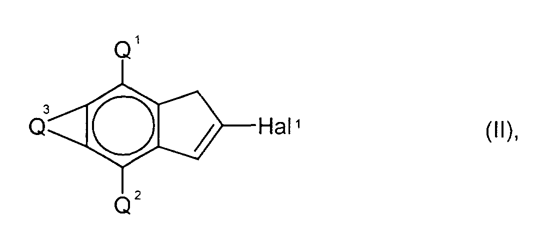 in der
Hall, Q¹, Q² und Q³ die in Anspruch 1 genannte Bedeutung haben,
mit einem elementaren Metall ausgewählt aus der 1., 2. oder 12. Gruppe des Periodensystems der Elemente nach IUPAC 1985 oder einer entsprechenden Metallverbindung in einer Menge im Bereich von 1 bis 100 Mol Metall/Metallverbindung pro Mol (II) und mit einem Dihalogenid von Y der Formel
Hal²-Y-Hal³ (III),
in der
Y, Hal² und Hal³ die in Anspruch 1 genannte Bedeutung haben,
in einer Menge von 1 bis 20 Mol (III) pro Mol (II) umsetzt, wobei in dem Fall, dass Y die Bedeutung -Si(R¹R²)-, -Ge(R¹R²)- oder -Si(R¹R²)-Si(R³R⁴)-hat, die Umsetzung von (II) mit (i) elementarem Metall/Metallverbindung und (ii) mit (III) auch simultan erfolgen kann.

12. Verfahren zur Herstellung der Zwischenprodukte der Formel (II) aus Anspruch 11, das dadurch gekennzeichnet ist, dass man ein gegebenenfalls substituierte Indanon der Formel 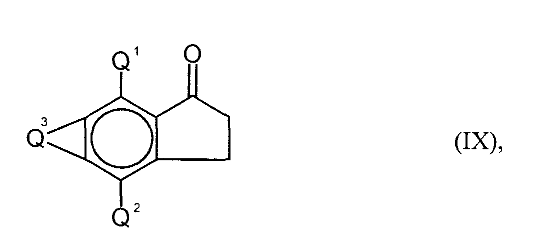 durch Umsetzung der aromatischen Verbindung der Formel 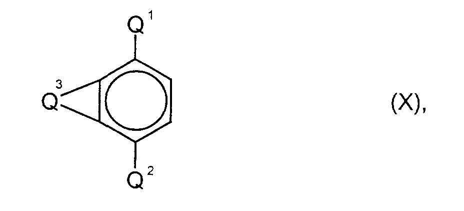 mit einem Acrylsäurederivat der Formel 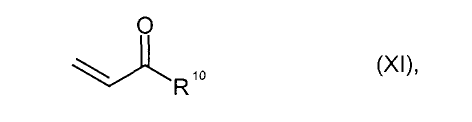 wobei
R¹⁰ Cl, Br, I, eine Hydroxygruppe oder eine C₁-C₁₀-Alkoxygruppe bedeutet,
in Gegenwart einer Lewissäure herstellt,
anschließend zu einem in 5,6-Position anellierten Inden der Formel 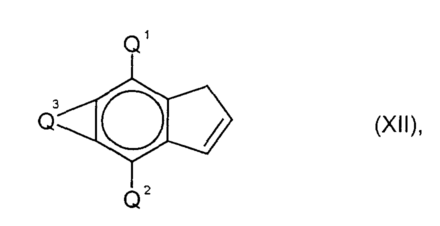 umsetzt,
und dann weiter in das Dihalogenderivat (XIII) überführt, 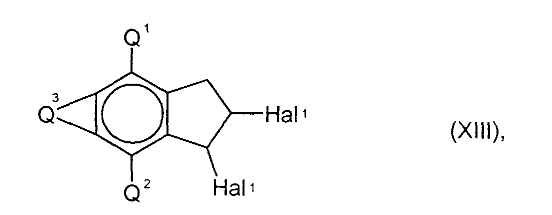 und nachfolgend eine Halogenwasserstoffabspaltung vornimmt,
wobei Hall, Q¹, Q² und Q³ die in Anspruch 1 genannte Bedeutung haben.

13. Verwendung der Verbindungen nach Anspruch 7 als Katalysatoren zur Polymerisation von Monomeren aus der Gruppe von C₂-C₁₂-α-Olefinen, C₄-C₂₀-Diolefinen und Cyclo(di)olefinen oder zur Copolymerisation mehrerer der genannten Monomeren.

14. Verwendung nach Anspruch 13 zur Herstellung amorpher, weitgehend ataktischer Polymerer.

15. Verwendung der Verbindung nach Anspruch 9 zur Herstellung von EP(D)M.

16. Verwendung der Verbindung nach Anspruch 9 zur Herstellung von ataktischem Polypropylen.
